**Europäisches Patentamt**

**European Patent Office**

**Office européen des brevets**

(19)

(11) **EP 1 243 575 A2**

(12) **EUROPÄISCHE PATENTANMELDUNG**

(43) Veröffentlichungstag:
**25.09.2002 Patentblatt 2002/39**

(51) Int Cl.⁷: **C07C 67/14**, C07C 67/08,
C07C 69/63, C07D 487/04

(21) Anmeldenummer: **02006039.8**

(22) Anmeldetag: **16.03.2002**

(84) Benannte Vertragsstaaten:
**AT BE CH CY DE DK ES FI FR GB GR IE IT LI LU
MC NL PT SE TR**
Benannte Erstreckungsstaaten:
**AL LT LV MK RO SI**

(30) Priorität: **24.03.2001 DE 10114565**

(71) Anmelder: **Solvay Fluor und Derivate GmbH
30173 Hannover (DE)**

(72) Erfinder:
• **Braun, Max
30900 Wendemark (DE)**
• **Eichholz, Kerstin
30855 Langenhagen (DE)**

(74) Vertreter: **Fischer, Reiner
Solvay Pharmaceuticals GmbH
Hans-Böckler-Allee 20
30173 Hannover (DE)**

(54) **Esterherstellung in Anwesenheit von Oniumsalzen**

(57)    Ein verbessertes Verfahren zur Herstellung von Carbonsäureestern aus Säurechloriden, Säurebromiden oder Säuren und Alkoholen sieht vor, die Veresterung in Anwesenheit des Addukts (Oniumsalz) der entsprechenden Carbonsäure und 1,5-Diaza-bicyclo [4.3.0]non-2-en oder 1,8-Diaza-bicyclo[5.4.0]-undec-7-en durchzuführen. Alternativ kann man in Anwesenheit der entsprechenden Carbonsäure und eines Oniumsalzes der entsprechenden Carbonsäure arbeiten.

EP 1 243 575 A2

**Beschreibung**

Die Erfindung bezieht sich auf ein Verfahren zur Herstellung von Carbonsäureestern.

Carbonsäureester sind beispielsweise als Lösungsmittel brauchbar. Sie sind auch Zwischenprodukte in der chemischen Synthese.

Das US-Patent 5,405,991 offenbart die Herstellung von Estern der Trifluoressigsäure und der Chlordifluoressigsäure aus den Säurechloriden und dem entsprechenden Alkohol in Anwesenheit von "Onium"-Salzen der dem eingesetzten Carbonsäurechlorid entsprechenden Carbonsäure. Das deutsche Patent 197 32 031 offenbart die Herstellung von Estern der Trifluoressigsäure und der Chlordifluoressigsäure in Anwesenheit der entsprechenden "Onium"-Salze unter 2-Phasen-Bildung zwecks einfacher Abtrennung der Produkte.

Aufgabe der vorliegenden Erfindung ist es, ein verbessertes Verfahren zur Herstellung von Carbonsäureestern aus Alkoholen und Carbonsäuren, Carbonsäurechloriden oder Carbonsäurebromiden anzugeben. Diese Aufgabe wird durch das in den Ansprüchen angegebene Verfahren gelöst.

Das erfindungsgemäße Verfahren zur Herstellung von Carbonsäureestern aus Alkoholen und Carbonsäuren, Carbonsäurechloriden oder Carbonsäurebromiden sieht vor, daß man die Veresterung unter Vermeidung des Ausfällens von "Onium"-Salzen durchführt, indem man in Anwesenheit des Addukts ("Onium"-Salz) der entsprechenden Carbonsäure und 1,5-Diazabicyclo[4.3.0]-non-2-en ("DBN") oder 1,8-Diaza-bicyclo-[5.4.0]-undec-7-en ("DBU") arbeitet, oder indem man in Anwesenheit der entsprechenden Carbonsäure und eines "Onium"-Salzes der entsprechenden Carbonsäure arbeitet.

Bevorzugt geht man bei der erfindungsgemäßen Herstellung von Carbonsäureestern von Alkoholen und Carbonsäurechloriden aus. Der Vorteil des erfindungsgemäßen Verfahrens ist, daß das Ausfällen von Feststoffen (im wesentlichen Hydrohalogenidsalze der "Onium"-Kationen) verhindert wird. Ausgefallene Feststoffe sind bei der Umsetzung und Aufarbeitung der Reaktionsmischung unerwünscht. Der Begriff "Alkohole" umfaßt auch Thioalkohole, die Begriffe "Carbonsäuren, Carbonsäurechloride oder Carbonsäurebromide" umfassen auch die entsprechenden Thiocarbonsäuren bzw. Thiocarbonsäurederivate.

Das Verfahren wird anhand der bevorzugten Verwendung von Carbonsäurechloriden weiter erläutert.

Bevorzugt stellt man Carbonsäureester der allgemeinen Formel (I) oder (II) her,

$$R^1\text{-}C(O)\text{-}OR^2 \tag{I}$$

$$R^1\text{-}C(O)\text{-}CH_2\text{-}C(O)\text{-}OR^2 \tag{II}$$

worin $R^1$ für C1-C4-Alkyl oder durch mindestens durch 1 Halogenatom substituiertes C1-C4-Alkyl steht, und $R^2$ für C1-C4-Alkyl, durch mindestens 1 Halogenatom substituiertes C1-C4-Alkyl, Aryl oder Benzyl steht. $R^1$ kann auch Aryl bedeuten, z. B. Phenyl.

Besonders bevorzugt steht $R^1$ für Methyl, Ethyl oder durch mindestens 1 Halogenatom substituiertes Methyl oder Ethyl.

Ganz besonders bevorzugt verestert man C1-C4-Alkohole und Säurechloride von halogenierten Essigsäuren und halogenierten Acetylessigsäuren. Ganz besonders bevorzugt stellt man entsprechende Alkylester der Chlor-, Dichlor-, Trichlor-, Trifluor-, Difluor- oder Chlordifluoressigsäure her oder entsprechende Alkylester der Trifluor-, Difluor- oder Chlordifluoracetylessigsäure, insbesondere der Trifluoracetylessigsäure.

Die Erfindung kann gemäß zweier Varianten durchgeführt werden. Bei der ersten Variante verwendet man die "Onium"-Salze zweier spezieller Amine, des DBN bzw. des DBU. Hier wird keine zusätzliche Säure eingesetzt. Bei der zweiten Variante wird in Anwesenheit freier Carbonsäure umgesetzt. Bei dieser Variante können beliebige stickstoffbasierte "Onium"-Salze eingesetzt werden, also auch von DBN und DBU. Die zweite Variante wird als erste näher erläutert.

Gemäß einer Variante führt man die Veresterung also in Anwesenheit eines "Onium"-Salzes der entsprechenden Säure (vorzugsweise einer halogenierten Essigsäure oder der halogenierten Acetylessigsäure) und zusätzlich der entsprechenden (freien) Säure (vorzugsweise einer halogenierten Essigsäure oder der (freien) halogenierten Acetylessigsäure) durch. Es ist also sowohl ein entsprechendes "Onium"-Salz als auch die freie Säure in der Mischung anwesend. Es wird angenommen, daß ein Teil der entsprechenden halogenierten Säure durch eine andere Carbonsäure oder Mineralsäure ersetzt werden kann. Es ist aber bevorzugt, wenn das Säurechlorid und die zur Bildung des "Onium"-Salzes verwendete Säure gleich sind.

Das Molverhältnis von "Onium"-Salz zu (freier) Carbonsäure liegt vorteilhaft zwischen 1:0,2 und 1:3. Gute Ergebnisse werden beispielsweise auch bei einem Verhältnis zwischen 1:0,2 und 1:2 erzielt. Die Konzentration an "Onium"-Salz beträgt vorteilhaft 5 bis 20 Mol.-% der Reaktanten (ohne Lösungsmittel berechnet).

Die Anwesenheit der freien Carbonsäure kann man durch ihre Zugabe zur Reaktionsmischung bewirken. Man kann sie auch in situ durch Zugabe von Wasser zur Reaktionsmischung erzeugen, weil das Säurechlorid und Wasser unter Bildung der entsprechenden freien Carbonsäure reagieren.

Das Verfahren kann kontinuierlich durchgeführt werden.

Der Begriff "Onium" steht in dieser Variante für Kationen mit positiv geladenem Stickstoff, beispielsweise protonierte aromatische Stickstoffbasen wie Pyridinium oder protonierte Alkyl-, Dialkyl- oder Trialkylammonium-Kationen oder für durch Cycloalkyl substituierte Ammonium-Verbindungen oder cycloaliphatische Stickstoffbasen wie Piperidinium oder quartäre Ammonium-Kationen.

Sehr gut geeignet als Carbonsäuresalz sind "Onium"-Salze, wobei "Onium" für ein Kation des Stickstoffs der Formel $R'R''R'''R''''N^+$ steht. $R'$, $R''$, $R'''$ und $R''''$ stehen unabhängig voneinander für Wasserstoff, Alkyl mit 1 bis 20 C-Atomen, Aryl oder Aralkyl. $R'$ und $R''$ oder $R'''$ und $R''''$, oder $R'$, $R''$ und $R'''$ oder $R'$, $R''$, $R'''$ und $R''''$ können auch, gegebenenfalls unter Einschluß des Stickstoff-Atoms, gesättigte oder ungesättigte Ringsysteme bilden. "Aryl" bedeutet hier insbesondere Phenyl oder durch 1 oder mehrere C1-C2-Alkylgruppen substituiertes Phenyl. Hervorragend geeignet sind Salze, in denen "Onium" für Ammonium, Pyridinium oder $R^{1'}R^{2'}R^{3'}R^{4'}N^+$ steht, worin $R^{1'}$, $R^{2'}$, $R^{3'}$ und $R^{4'}$ unabhängig voneinander Wasserstoff, Alkyl mit 1 bis 15 C-Atomen, Phenyl oder Benzyl stehen. Als Beispiel für solche Kationen seien genannt Pyridinium, Piperidinium, N-Methylpiperidinium, Anilinium, Benzyltriethylammonium und Triethylammonium.

Brauchbar sind auch durch Hydroxygruppen substituierte Amine, besonders cycloaliphatische Amine, insbesondere hydroxysubstituierte Piperidine und N-C1-C4-Alkylpiperidine. Geeignet sind z. B. die am C4-Atom substituierten Piperidine wie 4-Hydroxypiperidin, N-Methyl-4-hydröxypiperidin, N-Ethyl-4-hydroxypiperidin und N-Propyl-4-hydroxypiperidin.

Brauchbar sind auch Kationen von Aminen, welche in der nicht vorveröffentlichten deutschen Offenlegungsschrift ...(101 04 663.4) offenbart sind. Es handelt sich um "Onium"-Kationen auf Basis einer mono- oder bicyclischen Verbindung mit mindestens 2 Stickstoffatomen, wobei mindestens 1 Stickstoffatom in das Ringsystem eingebaut ist.

So kann man "Onium"-Kationen auf Basis von monocyclischen Verbindungen einsetzen. Es handelt sich dann um gesättigte oder ungesättigte 5-Ring-, 6-Ring- oder 7-Ring-Verbindungen. Mindestens 1 Stickstoffatom ist in den Ring eingebaut. Es kann auch noch ein weiteres Stickstoffatom in das Ringsystem eingebaut sein. Alternativ oder zusätzlich kann der Ring durch eine oder mehrere Aminogruppen substituiert sein. Bevorzugt sind Dialkylaminogruppen, in denen die Alkylgruppen gleich oder verschieden sein können und 1 bis 4 Kohlenstoffatome umfassen. Die Aminogruppe kann auch ein gesättigtes Ringsystem, beispielsweise eine Piperidinogruppe, darstellen. Gut brauchbare Vertreter von monocyclischen Ringsystemen sind Dialkylaminopyridin, Dialkylaminopiperidin und Dialkylaminopiperazin.

Auch "Onium"-Kationen bicyclischer Verbindungen kann man einsetzen. Auch hier können 1, 2 oder mehr Stickstoffatome in das Ringsystem integriert sein. Die Verbindungen können durch eine oder mehr Aminogruppen substituiert sein. Bevorzugt sind wieder Dialkylaminogruppen, wobei die Alkylgruppen gleich oder verschieden sein können und 1 bis 4 C-Atome umfassen oder zusammen mit dem Stickstoffatom ein gesättigtes Ringsystem bilden, wie beispielsweise die Piperidinyl-Gruppe.

Aus dem vorstehend gesagten wird klar, daß bei dieser Ausführungsform mindestens 2 Stickstoffatome in den brauchbaren Verbindungen basische Eigenschaften aufweisen müssen und, je nach Art der Bindungen, an 2 oder 3 Kohlenstoffatome gebunden sind.

Ganz besonders bevorzugt sind "Onium"-Salze der Carbonsäure mit bicyclischen Amidinen, insbesondere 1,5-Diazabicyclo[4.3.0]non-5-en (DBN) und 1,8-Diazabicyclo[5.4.0]-undec-7-cen (DBU). Man kann diese Salze auch gemeinsam mit freier Carbonsäure einsetzen, wie dies mit den obenangegebenen "Onium"-Salzen möglich ist.

Eine andere Variante der Erfindung sieht vor, daß man in Anwesenheit des Addukts ("Onium"-Salz) der entsprechenden Carbonsäure und 1,5-Diazabicyclo-[4.3.0]-non-2-en oder 1,8-Diaza-bicyclo-[5.4.0]-undec-7-en arbeitet. Bei dieser Variante arbeitet man ohne zusätzliche freie Säure.

Die vorstehend genannten "Onium"-Verbindungen kann man vorab herstellen, indem man die Amine mit der jeweiligen Säure umsetzt.

Die Temperatur, bei der die Umsetzung im erfindungsgemäßen Verfahren durchgeführt wird, liegt bei Umgebungstemperatur (etwa 20 °C) bis hin zum Siedepunkt der Mischung, beispielsweise bis hin zu 100 °C. Man arbeitet bei Umgebungsdruck (etwa 1 bar abs.) oder gewünschtenfalls auch bei erhöhtem Druck, beispielsweise bei einem Druck von bis zu 5 bar abs.

Das Verfahren kann batchweise oder kontinuierlich durchgeführt werden. Die Aufarbeitung zwecks Gewinnung der gebildeten Ester kann beispielsweise durch Destillation erfolgen.

In einer bevorzugten Ausführungsform nutzt man die Bildung zweier Phasen aus. Dies kann beispielsweise bewirkt werden, indem das Reaktionsgemisch auf tiefe Temperaturen abgekühlt wird. Eine Phase stellt die oft schon sehr reine Esterphase dar.

Bei anderen Estern findet eine Phasentrennung bereits bei Umgebungstemperatur statt. Dies gilt beispielsweise bei den im deutschen Patent 197 32 031 offenbarten Methyl- und Ethylestern der Trifluoressigsäure bzw. Chlordifluoressigsäure. Zweckmäßig stellt man ein Molverhältnis von Alkohol zu Säurechlorid, wie in der angegebenen deutschen Patentschrift beschrieben, im Bereich von 1,01:1 bis 5:1 ein. Bevorzugt beträgt das Molverhältnis von Methanol zu Trifluoracetylchlorid etwa 1,03:1 bis 4:1, das Molverhältnis von Ethanol zu Trifluoracetylchlorid etwa 1,01:1 bis 5:1, das Molverhältnis von Methanol zu Chlordifluoracetylchlorid etwa 1,06:1 bis 2,5:1 und das Molverhältnis von Ethanol zu Chlordifluoracetylchlorid etwa 1,02:1 bis 2,5:1.

Bei der erfindungsgemäßen Herstellung wird bei Verwendung von Säurechloriden HCl freigesetzt. Die freigesetzte HCl kann mit Alkohol unter Alkylchloridbildung reagieren. Es hat sich gezeigt, daß die Alkylchloridbildung weitgehend unterdrückt werden kann, wenn man nichtaromatische "Onium"-Salze einsetzt, insbesondere Trialkylammoniumverbindungen; vorteilhaft gelingt die Unterdrückung der Alkylchloridbildung auch, insbesondere bei kontinuierlicher Verfahrensweise und Abnehmen einer 2-Phasen-Mischung aus dem Sumpf des Reaktors, durch Rückspeisen der abgetrennten Katalysatorphase (die ja "Onium"-Carbonsäuresalz enthält) in diejenigen Bauteile des Reaktors, in welchen Alkohol und HCl in Kontakt miteinander stehen, beispielsweise im Stripper oder diejenigen Bauteile, in welchen verdampfende Bestandteile der Reaktionsmischung unter Rückfluß kondensiert werden.

Ein weiterer Gegenstand der Erfindung sind neue "Onium"-Salze, die als Katalysator im erfindungsgemäßen Veresterungsverfahren brauchbar sind. Es handelt sich um Salze, die aus Carbonsäureanionen und "Onium"-Kationen auf Basis einer monooder bicyclischen Verbindung mit mindestens 2 Stickstoffatomen, wobei mindestens 1 Stickstoffatom in das Ringsystem eingebaut ist, gebildet sind. Die bevorzugten Carbonsäureanionen sind diejenigen, die den Carbonsäureestern der allgemeinen Formel (I) und (II), wie weiter oben angegeben, entsprechen. Besonders bevorzugt sind Carbonsäureanionen, die den weiter oben als bevorzugt geschilderten Carbonsäureestern entsprechen. Brauchbare und bevorzugte "Onium"-Kationen sind weiter oben angegeben. Besonders bevorzugt sind "Onium"-Salze gebildet aus Dialkylaminopyridinium-, Dialkylaminopiperidinium-, Dialkylaminopiperazinium-, der obengenannten hydroxysubstituierten Piperidinium-Kationen, protonierten Kationen von DBN und DBU als Kationen und Trifluoracetat, Difluoracetat, Chlordifluoracetat, Trifluoracetylacetat, Chlordifluoracetylacetat und Difluoracetylacetat. Ganz besonders bevorzugt sind Salze gebildet aus protonierten Kationen von DBN und DBU und Trifluoracetat. Die genannten Salze können als Katalysator im erfindungsgemäßen Verfahren eingesetzt werden.

Noch ein Gegenstand der Erfindung sind Gemische, die Carbonsäuren und auf Stickstoff basierende Salze aus "Onium"-Kationen und Carbonsäuranionen, vorzugsweise entsprechend der im Gemisch vorliegenden Carbonsäure, umfassen. Das Molverhältnis von "Onium"-Salz zu Carbonsäure liegt im Bereich von 1:0,2 bis 1:3. Bevorzugte "Onium"-Kationen (sei es mit einem oder mit mindestens zwei Stickstoffatomen) sind weiter oben angegeben. Bevorzugte Carbonsäureanionen sind ebenfalls vorstehend angegeben. Die erfindungsgemäßen Gemische aus "Onium"-Salzen und Carbonsäure sind ebenfalls als Katalysator im erfindungsgemäßen Veresterungsverfahren brauchbar. Vorteil des erfindungsgemäßen Verfahrens ist bessere Handhabung, da das Ausfällen von Feststoffen vermieden wird, und die mögliche verringerte Bildung von Nebenprodukten, insbesondere die verringerte Alkylchloridbildung bei Einsatz von Carbonsäurechloriden in der Veresterung.

Die folgenden Beispiele sollen die Erfindung weiter erläutern, ohne sie in ihrem Umfang einzuschränken.

**Herstellung von ethylchloridfreiem Trifluoressigsäureethylester**

**Beispiel 1:**

Einsatz von Oniumacetat, Trifluoressigsäure und Wasser

Ansatz:

| | |
|---|---|
| 1,0 mol Ethanol | 32,05 |
| 0,1 mol Trifluoressigsäure | 11,40 |
| 0,1 mol Triethylamin | 10,10 |
| 1,0 mol Trifluoracetylchlorid | 132,47 |
| 0,025 mol Wasser | 0,45 |

Aufbau u. Durchführung:

In einem 250 ml 3-Halskolben wurde der Alkohol vorgelegt und das Amin eingewogen, dann wurde sehr vorsichtig die Trifluoressigsäure zugetropft. Danach wurde noch das Wasser zugegeben, um ein 2-Phasensystem zu realisieren. Der Kolben wurde mit einem Rückflußkühler (-40 °C) versehen und in einem Ölbad auf ca. 55 °C temperiert. Bei dieser Temperatur wurde unter Rühren TFAC eingeleitet. Zwischen 27 und 30 g TFAC (~20 mol-%) trübte sich die Lösung ein und bildete eine zweite Phase aus (die obere Phase enthielt den Katalysator). Probe 01 wurde aus der flüssigen Esterphase, Probe 02 aus der über der Reaktionsmischung stehenden Gasphase genommen.

Bei 70,1 g TFAC war die obere Phase nur noch ca. 2 mm dick. Ihr wurde Probe 03 entnommen (Esterphase). Bei 80 g (~60 mol-%) war dann nur noch eine Phase vorhanden.

Ergebnis (Analysen jeweils in GC Flächenprozent):

| Probe | % HCl | %TFAC | %EtCl | %EtOH | %TFAEt |
|-------|-------|-------|-------|-------|--------|
| 01 | 1,22 | 0,31 | | 5,70 | 92,32 |
| 02 | 48,61 | | 0,69 | 0,17 | 50,08 |
| 03 | 3,97 | 0,77 | | 1,23 | 87,31 |

Ethylchlorid konnte nur in der Gasphase nachgewiesen werden.

**Beispiel 2:**

Verwendung von DBN

Ansatz:

| 1,0 mol Ethanol | 32,05 |
|---|---|
| 0,1 mol Trifluoressigsäure | 11,40 |
| 0,1 mol 1,5-Diazabicyclo[4.3.0]non-5-en | 12,40 |
| 1,0 mol Trifluoracetylchlorid | 132,47 |

Aufbau u. Durchführung:

In einem 250 ml 3-Halskolben wurde der Alkohol vorgelegt und das Amin eingewogen, dann wurde sehr vorsichtig die Trifluoressigsäure zugetropft. DBN reagierte sehr viel heftiger mit der TFA als z. B. Pyridin. Der Kolben wurde mit einem Rückflußkühler (-40 °C) versehen und in einem Ölbad auf ca. 55 °C temperiert. Bei dieser Temperatur wurde unter Rühren TFAC eingeleitet.

Bei ca. 33 g TFAC (~20 mol-%) bezogen auf die eingesetzte Ethanolmenge trübte sich die Lösung ein, bei 40 g bildete sich eine klare, zweite Phase aus (die obere Phase war die Esterphase). Probe 01 wurde aus der flüssigen Esterphase, Probe 02 aus der über der Reaktionsmischung stehenden Gasphase genommen. Bei 88 g (~68 mol-%) war nur noch eine Phase vorhanden.

Ergebnis:

| Probe | % HCl | %TFAC | %EtCl | %EtOH | %TFAEt |
|-------|-------|-------|-------|-------|--------|
| 01 | 1,70 | 1,31 | | | 96,19 |
| 02 | 62,67 | 0,72 | 0,15 | 0,09 | 29,32 |
| 03 | | 0,77 | 0,03 | 0,04 | 98,99 |

Probe 03 wurde erst nach dem Einleiten der vollständigen TFAC-Menge genommen und ist durch Hydrolyse aufgearbeitet.

**Beispiel 3:**

Verwendung von 4-Hydroxy-N-methylpiperidin

Ansatz:

| | |
|---|---|
| 1,0 mol Ethanol | 32,05 |
| 0,1 mol Trifluoressigsäure | 11,40 |
| 0,1 mol 4-Hydroxy-N-methylpiperidin | 11,50 |
| 1,0 mol Trifluoracetylchlorid | 132,47 |
| 0,093 mol Wasser | 1,68 |

Aufbau u. Durchführung:

In einem 250 ml 3-Halskolben wurde der Alkohol vorgelegt und das Amin eingewogen. Dann wurde sehr vorsichtig die Trifluoressigsäure zugetropft. Danach wurde noch das Wasser zugegeben, um ein 2-Phasensystem zu realisieren. 4-Hydroxy-N-methylpiperidin reagierte nicht so heftig mit der TFA wie z. B. DBN. Der Kolben wurde mit einem Rückflußkühler (-40 °C) versehen und in einem Ölbad auf ca. 55 °C temperiert. Bei dieser Temperatur wurde unter Rühren TFAC eingeleitet. Bei ca. 40 g TFAC (~30 mol-%) trübte sich die Lösung ein und bildete eine klare, zweite Phase aus (das "Onium"-Salz bildete die obere Phase). Probenahme 01.
Bei 78 g (~60 mol-%) lagen immer noch 2 Phasen vor,(Proben 02/03) einzelne "Fettaugen", wie dicke Perlen. Bei weiterer Dosierung fielen die "Katalysatorperlen" nach unten , 2 Phasen blieben bestehen, aber TFA und Amin diffundierten in die Esterphase.

Ergebnis:

| Probe | % HCl | %EtOH | %TFAEt | %TFA | % Amin |
|-------|-------|-------|--------|------|--------|
| 01 | 0,08 | 4,43 | 94,51 | | |
| 02 | 0,88 | 0,37 | 98,14 | | |
| 03 | 55,39 | | 37,51 | | |
| 04 | 0,73 | 0,19 | 86,17 | 7,49 | 5,39 |

Probe 04 wurde erst nach dem Einleiten 1:1 genommen, hydrolysiert.

Probe 01, 02 und 04 wurden aus der flüssigen Esterphase, Probe 03 aus der über der Reaktionsmischung stehenden Gasphase genommen.

**Beispiel 4:**

Ansatz:

| | |
|---|---|
| 1,0 mol Ethanol | 32,05 |
| 0,1 mol Trifluoressigsäure | 11,40 |
| 0,1 mol N- Methylpiperidin | 9,92 |
| 1,0 mol Trifluoracetylchlorid | 132,47 |
| 0,025 mol Wasser | 0,45 |

Aufbau u. Durchführung:

In einem 250 ml 3-Halskolben wurde der Alkohol vorgelegt und das Amin eingewogen. Dann wurde sehr vorsichtig die Trifluoressigsäure zugetropft. Danach wurde noch das Wasser zugegeben, um ein 2-Phasensystem zu realisieren. N-Methylpiperidin reagierte nicht so heftig mit der TFA wie z. B. DBN. Der Kolben wurde mit einem Trokkeneiskühler (-70 °C) versehen und in einem Ölbad auf ca. 55 °C temperiert. Bei dieser Temperatur wurde unter Rühren TFAC eingeleitet.

Bei ca. 30 g TFAC (~23 mol-%) trübte sich die Lösung ein und bildete eine klare, zweite Phase aus ( Katalysatorphase oben). Probenahme 01/02, die Probe 01 wurde nach ca. 1 h noch einmal analysiert, da sich die Katphase noch nicht vollständig separiert hatte (dies war Probe 03).

Bei 89 g (~67 mol-%) lagen immer noch 2 Phasen vor, Probe 04. Phasenvereinigung erfolgte bei ca. 72 mol-%.

Ergebnis:

| Probe | % HCl | %EtCl | %EtOH | %TFAEt | %TFA | % Amin |
|---|---|---|---|---|---|---|
| 01 | 1,60 | | 10,01 | 81,58 | 1,95 | 4,46 |
| 02 | 40,36 | 0,487 | 0,44 | 42,85 | | |
| 03 | 0,73 | | 6,29 | 91,15 | 1,12 | 0,66 |
| 04 | 4,29 | | 0,78 | 78,22 | 7,49 | 8,24 |

Probe 01 und 03 wurden aus der flüssigen Esterphase, Probe 02 aus der über der Reaktionsmischung stehenden Gasphase genommen; Probe 04 nach Vereinigen der Phasen zu Reaktionsende.

**Beispiel 5:**

Verwendung der DBU

Ansatz:

| | |
|---|---|
| 1,0 mol Ethanol | 32,05 |
| 0,2 mol Trifluoressigsäure | 22,80 |

(fortgesetzt)

| | |
|---|---|
| 0,1 mol 1,8 Diazabicyclo[5.4.0]undec-7-en | 15,20 g |
| 0,5 mol Trifluoracetylchlorid | 66,2 g |
| 0,1 mol Wasser | 1,8 g |

Aufbau u. Durchführung:

In einem 250 ml 3-Halskolben wurde der Alkohol vorgelegt und das Amin eingewogen. Dann wurde sehr vorsichtig die Trifluoressigsäure zugetropft. DBU reagierte sehr viel heftiger mit der TFA als z. B. Pyridin. Der Kolben wurde mit einem Rückflußkühler (-40 °C) versehen und in einem Ölbad auf ca. 55 °C temperiert. Bei dieser Temperatur wurde unter Rühren TFAC eingeleitet.

Bei ca. 13 g TFAC (~10 mol-%) trübte sich die Lösung ein, und dicke gelbe Flocken fielen aus (Probenahme 01).

Bei 88 g (~68 mol-%) war nur noch eine Phase vorhanden. Nach Zugabe eines weiteren Äquivalentes TFA löste sich der Niederschlag auf. Entnahme von Probe 02.

Bei weiterem Einleiten von TFAC fiel der Niederschlag wieder aus, daraufhin wurden noch 0,1 mol Wasser zugefügt und nach 1 h Stehenlassen Probe 03 aus der entstandenen Esterphase entnommen.

Ergebnis:

| Probe | % HCl | %EtCl | %EtOH | %TFAEt | %TFA |
|---|---|---|---|---|---|
| 01 | 0,91 | | 4,63 | 91,55 | 2,08 |
| 02 | 49,86 | 1,51 | 0,09 | 44,67 | |
| 03 | 0,34 | | 0,41 | 98,76 | |

Proben 01 und 03 wurden aus der flüssigen Esterphase, Probe 02 aus der über der Reaktionsmischung stehenden Gasphase genommen.

**Beispiel 6:**

Verwendung eines Überschusses aus Trifluoressigsäure

Ansatz:

| | |
|---|---|
| 1,0 mol Ethanol | 32,05 g |
| 0,25 mol Trifluoressigsäure | 28,50 g |
| 0,1 mol Piperidin | 8,50 g |
| 1,0 mol Trifluoracetylchlorid | 132,47 g |

Aufbau u. Durchführung:

In einem 250 ml 3-Halskolben wurde der Alkohol vorgelegt und das Amin eingewogen. Dann wurde sehr vorsichtig die 0,1 mol Trifluoressigsäure zugetropft. Der Kolben wurde mit einem Rückflußkühler (-40 °C) versehen und bei Raumtemperatur wurde unter Rühren TFAC eingeleitet, so das eine Temperatur von 30 °C nicht überschritten wurde. Bei ca. 1,3 g TFAC trübte sich die Lösung ein und Kristalle fielen aus. Nach 23,4 g wurde die Einleitung abgebrochen, weil der Kolbeninhalt festgeworden war, und ein auf 70 °C temperiertes Ölbad unter den Kolben gestellt. Bei dieser Temperatur wurde die restliche TFA (ca. 18 g) zugegeben. Das Salz löste sich auf, bildete jedoch zunächst keine zweite Phase. Nach Einleiten von 50 % der o. g. TFAC-Menge erfolgte dann die Phasentrennung. Probe 01 wurde genommen.

Bei ca. 80 % wurde Probe 02 aus der Gasphase vor dem Kühler genommen; Probe 04 stammte aus der Gasphase nach dem Kühler, Probe 03 gab die Zusammensetzung der Esterphase zu diesem Zeitpunkt wieder.

Nach weiteren TFAC-Einleiten erfolgte die Phasenvermischung, d. h. das Verschwinden des Zweiphasensystems nach Einleiten von ca. 85 % der TFAC Menge. Nach Phasenvermischung wurde die Probe 05 genommen (hydrolysiert).

Ergebnis:

| Probe | % HCl | %TFAC | %EtCl | %EtOH | %TFAEt | %TFA |
|-------|-------|-------|-------|-------|--------|------|
| 01 | | | 0,03 | 0,41 | 99,33 | |
| 02 | 58,90 | 7,3 | 0,16 | | 31,53 | 1,52 |
| 03 | 5,40 | | 0,018 | 0,22 | 87,17 | 6,33 |
| 04 | 97,53 | | 0,10 | | 1,75 | |
| 05 | | | 0,02 | 0,073 | 99,59 | |

Proben 01 und 03 wurden aus der flüssigen Esterphase, Probe 02 aus der über der Reaktionsmischung stehenden Gasphase genommen, Probe 04 nach dem aufgesetzten Kühler.

**Beispiel 7:**

Herstellung von Trifluoressigsäureethylester (TFAEt) mit DBN x 3 TFA

Reaktionsgleichung:

$$C_2H_5OH + CF_3COCl \rightarrow CF_3CO_2Et + HCl$$

Ansatz:

| | |
|---|---|
| 46,07 g (1,0 mol) | Ethanol |
| 34,20 g (0,3 mol) | Trifluoressigsäure (TFA) |
| 12,40 g (0,1 mol) | DBN |
| 132,47 g (1,0 mol) | Trifluoracetylchlorid (TFAC) |

Aufbau und Durchführung:

In einem 250 ml Dreihalskolben wurde der Alkohol und das Amin eingewogen. Dann wurde sehr vorsichtig die TFA zugetropft. Das DBN reagierte sehr heftig mit der TFA. Der Kolben wurde mit einem Trockeneiskühler versehen und im Ölbad auf ca. 50 °C temperiert. Bei erreichter Temperatur wurde unter kräftigem Rühren TFAC eingeleitet. Bei ca. 40 g TFAC ≈ 30 mol-% trübte sich die Lösung. Bei 42 g bildete sich eine zweite, klare Phase aus (Katphase oben).

Bei 94 mol-% ≈ 125 g TFAC war nur noch eine Phase vorhanden (gelblich, klar). Die Reinheit des TFAEt im Zweiphasengebiet in der organischen Phase betrug ohne weitere Aufreinigung schon 98 %. Der Umsatz zu TFAEt war quantitativ.

Das Beispiel belegt, daß die Umsetzung auch unter Verwendung des Addukts von DBN und Trifluoressigsäure mit zusätzlicher freier Säure funktioniert.

**Beispiel 8:**

Herstellung von methylchloridfreiem Trifluoressigsäuremethylester (TFAMe) mit DBN x 3 TFA

Reaktionsgleichung:

$$CH_3OH + CF_3COCl \rightarrow CF_3CO_2CH_3 + HCl$$

Ansatz:

| | |
|---|---|
| 32,04 g (1,0 mol) | Methanol |
| 34,20 g (0,3 mol) | Trifluoressigsäure |
| 12,40 g (0,1 mol) | DBN |
| 132,47 g (1,0 mol) | TFAC |

Aufbau und Durchführung:

In einem 250 ml Dreihalskolben wurde der Alkohol und das Amin eingewogen. Dann wurde sehr vorsichtig die TFA zugetropft. Das DBN reagierte sehr heftig mit der TFA. Der Kolben wurde mit einem Trockeneiskühler versehen und im Ölbad auf ca. 50 °C temperiert. Bei erreichter Temperatur wurde unter kräftigem Rühren TFAC eingeleitet.
Bei ca. 26 g TFAC ≈ 20 mol-% trübte sich die Lösung. Bei 28 g bildete sich eine zweite, klare Phase aus (Katphase oben).
Bei 92 mol-% ≈ 121 g TFAC war nur noch eine Phase vorhanden (gelblich, klar). Die Reinheit des TFAMe im Zweiphasengebiet in der organischen Phase betrug ohne weitere Aufreinigung schon 99 %. Der Umsatz zu TFAMe war quantitativ.
Das Beispiel belegt, daß auch bei Verwendung des Addukts von DBN und Trifluoressigsäure mit zusätzlicher freier Säure methylchloridfreies Produkt erhalten wird.

**Patentansprüche**

1. Verfahren zur Herstellung von Carbonsäureestern aus Alkoholen und Carbonsäuren, Carbonsäurechloriden oder Carbonsäurebromiden, wobei man die Veresterung unter Vermeidung des Ausfällens von "Onium"-Salzen durchführt, indem man
in Anwesenheit des Addukts (Oniumsalz) der entsprechenden Carbonsäure und 1,5-Diaza-bicyclo[4.3.0]-non-2-en oder 1,8-Diaza-bicyclo[5.4.0]-undec-7-en arbeitet, oder
wobei man in Anwesenheit der entsprechenden Carbonsäure und eines Oniumsalzes der entsprechenden Carbonsäure arbeitet.

2. Verfahren nach Anspruch 1, **dadurch gekennzeichnet, daß** man im 2-Phasengebiet arbeitet (Flüssig-Flüssig-Phase).

3. Verfahren nach Anspruch 1, **dadurch gekennzeichnet, daß** man Carbonsäureester der allgemeinen Formel (I) oder (II)

$$R^1\text{-C(O)-O}R^2 \tag{I}$$

$$R^1\text{-C(O)-CH}_2\text{-C(O)-O}R^2 \tag{II}$$

herstellt, worin R$^1$ für Aryl, C1-C4-Alkyl oder durch mindestens durch 1 Halogenatom substituiertes C1-C4-Alkyl steht, und R$^2$ für C1-C4-Alkyl, durch mindestens 1 Halogenatom substituiertes C1-C4-Alkyl, Aryl oder Benzyl steht.

4. Verfahren nach Anspruch 3, **dadurch gekennzeichnet, daß** R$^1$ für Methyl, Ethyl oder durch mindestens durch 1 Halogenatom substituiertes Methyl oder Ethyl steht.

5. Verfahren nach Anspruch 1, **dadurch gekennzeichnet, daß** man von Säurechloriden ausgeht.

6. Verfahren nach Anspruch 5 zur Herstellung von Alkylestern von halogenierten Essigsäuren und halogenierten Acetylessigsäuren aus den Säurechloriden und Alkylalkoholen in Anwesenheit von "Onium"-Salzen der entsprechenden halogenierten Essigsäure oder halogenierten Acetylessigsäure, wobei man in Anwesenheit des Addukts der halogenierten Essigsäure oder halogenierten Acetylessigsäure und 1,5-Diaza-bicyclo-[4.3.0]-non-5-en oder 1,8-Diaza-bicyclo[5.4.0]-undec-7-en als "Onium"-Salz arbeitet, oder wobei man in Anwesenheit eines "Onium"-Salzes der entsprechenden halogenierten Essigsäure oder der halogenierten Acetylessigsäure und der entsprechenden halogenierten Essigsäure oder der halogenierten Acetylessigsäure arbeitet.

7. Verfahren nach Anspruch 6, **dadurch gekennzeichnet, daß** man die halogenierte Essigsäure oder halogenierte Acetylessigsäure in situ durch Zugabe von Wasser zur Reaktionsmischung erzeugt.

8. Verfahren nach Anspruch 6, **dadurch gekennzeichnet, daß** man Alkylester der Tri-, Difluor- oder Chlordifluoressigsäure oder der Trifluoracetylessigsäure herstellt.

9. Verfahren nach Anspruch 1, **dadurch gekennzeichnet, daß** das Molverhältnis zwischen Oniumsalz und Säure im Bereich von 1:0,2 bis 1:3 liegt.

10. Verfahren nach Anspruch 1, **dadurch gekennzeichnet, daß** man eine Verringerung der Alkylchloridbildung bewirkt, indem man nichtaromatische "Onium"-Salze einsetzt.

11. Verfahren nach Anspruch 1, **dadurch gekennzeichnet, daß** man ein Oniumsalz eines Trialkylamins einsetzt.

12. Verfahren nach Anspruch 1, **dadurch gekennzeichnet, daß** man ein Oniumsalz von Piperidin, N-Alkylpiperidin, Hydroxypiperidin oder N-Alkyl-Hydroxypiperidin einsetzt.

13. Verfahren nach einem der vorhergehenden Ansprüche, **dadurch gekennzeichnet, daß** man einen C1-C4-Alkylalkohol einsetzt.

14. Verfahren nach Anspruch 13, **dadurch gekennzeichnet, daß** man Methylalkohol oder Ethylalkohol einsetzt.

15. "Onium"-Salze auf Basis von Carbonsäuren und monooder bicyclischen Verbindungen mit mindestens 2 Stickstoffatomen, wobei mindestens 1 Stickstoffatom in das Ringsystem eingebaut ist.

16. Gemische, umfassend Carbonsäuren und N-basierte "Onium"-Salze von Carbonsäuren.